# EUROPEAN PATENT APPLICATION

(11) **EP 1 705 635 A2**
(43) Date of publication of application: **27.09.2006**
(21) Application number: 05258083.4
(22) Date of filing: 28.12.2005
(51) Int. Cl.: G09G 3/36, G09G 3/20

(54) **Image display device with appropriate subpixel driving based on discrimination of colour/black and white images and electronic apparatus including the same**

(30) Priority: 24.03.2005 JP 2005085564
(71) Applicant: Sanyo Epson Imaging Devices Corporation, Tokyo 105-6115 (JP)
(72) Inventor: Kurumisawa, Takashi Sanyo Epson Imaging Devices, Nagano 399-8285 (JP)
(74) Representative: Cloughley, Peter Andrew

(57) **Abstract**

An image display device is provided which includes a display panel in which each pixel region has four color sub-pixel regions, an image processing unit that generates color signals for the four color sub-pixels on the basis of an input image signal from the outside, and a control unit that display an image in the pixels of the display panel on the basis of the color signals. In the image display device, the image processing unit includes a discriminating unit that discriminates whether the input image signal is a black-and-white image or a color image for every predetermined unit of the input image signals, a black-and-white image processing unit that detects line segment parts forming line segments from the input image signal and generates color signals for a black-and-white image corresponding to the line segment parts when it is discriminated that the input image signal is a black-and-white image, and a color image processing unit that generates color signals for a color image when it is discriminated that the input image signal is a color image and when it is discriminated that the predetermined unit of the image data is not the line segment parts.

## Description

The present invention relates to an electro-optical device such as a liquid crystal device and to an electronic apparatus. Further, the invention relates to an electrophoresis device such as an electronic paper and an electroluminescent (EL) device.

Recently, color image display devices such as a color liquid crystal display device have been used to a portable terminal device such as a mobile phone, a PDA, etc. For example, a liquid crystal display device performs color display such that color filters are provided on one of a pair of transparent substrates with liquid crystal interposed therebetween. A general color filter is configured by repeatedly arranging red (R), green (G), and blue (B) filter regions according to an additive color mixing system. In particular, the red filter regions, the green filter regions, and the blue color filter regions are formed to be adjacent to one another, and one red filter region, one green filter region, and one blue filter region form one color pixel.

In a case that a color image display device using RGB color filters performs color display, colors that can be represented by R, G, and B colors are limited to colors in a region defined by a color triangle whose vertices are R, G, and B on a CIE chromaticity diagram.

Meanwhile, a method that forms one color pixel with sub-pixels of four colors containing one more color in addition to R, G, and B is disclosed in JP-A-3-109525. Further, a method of realizing color display and high-definition black-and-white display by using R, G, B, and white pixels is disclosed in JP-A-10-10517.

An advantage of some aspects of the invention is that it provides an image display device in which four color sub-pixel regions form one pixel and that can perform color image display with high color reproductivity and high-definition black-and-white image display by using a color input image signal such as a RGB signal.

According to a first aspect of the invention, an image display device is provided which includes a display panel in which each pixel region has four color sub-pixel regions, an image processing unit that generates color signals for the four color sub-pixels on the basis of an input image signal from the outside, and a control unit that display an image in the pixels of the display panel on the basis of the color signals. In the image display device, the image processing unit includes: a discriminating unit that discriminates whether the input image signal is a black-and-white image or a color image for every predetermined unit of the input image signals; a black-and-white image processing unit that detects line segment parts forming line segments from the input image signal and generates color signals for a black-and-white image corresponding to the line segment parts when it is discriminated that the input image signal is a black-and-white image; and a color image processing unit that generates color signals for a color image when it is discriminated that the input image signal is a color image and when it is discriminated that the predetermined unit of the image data is not the line segment parts.

In the image display device, the region of one color pixel is composed of sub-pixel regions of four colors (for example, R, G, and B, plus C (cyan) or W (white)). Color signals corresponding the sub-pixels of four colors are generated on the basis of an input image signal such as an RGB signal from the outside. In the above-mentioned example, RGBC or RGBW color signals are generated on the basis of an RGB input image signals. Then, display is performed on the sub-pixels of the display panel on the basis of the four-color signal. Therefore, it is possible to expand the color reproduction range in the color image display by using the sub-pixels of four colors (R, G, B, and another color) and rich color reproduction is possible.

In particular, it is discriminated whether an input image signal is a black-and-white image or a color image for every predetermined unit and line segment parts are detected from a black-and-white image. Then, a color signal for a black-and-white image corresponding to each line segment part is generated, and a black-and-white image is displayed on the basis of the color signals. Meanwhile, when it is discriminated that the input image signal is a color image, and when it is discriminated that the predetermined unit of image data of the input image signal is not a line segment part, a color signal for a color image is generated and color display is performed. Here, it is possible to compose more black-and-white pixels than the number of color pixels by combining sub-pixels of four colors constituting any color pixel with sub-pixels constituting any other neighboring color pixel. Also, when the image is a black-and-white (achromatic) image, the image is generally composed of line segments as characters, figures, etc. Therefore, when white display or black display is performed in black-and-white pixel units, it is possible to perform black-and-white image display with high resolution.

In the image display device according to the first aspect of the invention, the regions of the sub-pixels of four colors may be arranged in a vertical direction so as to form one vertical pixel region, and the black-and-white image processing unit may generate the color signals for a black-and-white image representing white or black in one pixel unit in the vertical direction. Alternately, the regions of the sub-pixels of four colors may be arranged in a horizontal direction so as to form one horizontal pixel region, and the black-and-white image processing unit may generate the color signals for a black-and-white image representing white or black in one pixel unit in the horizontal direction.

Further, in the image display device according to the first aspect of the invention, the discriminating unit may include a converting part that converts the input image signal into a luminance signal and a color difference signal for every predetermined unit, and a discriminating part that discriminates that the input image signal is a black-and-white image when the color difference signal is less than a predetermined value and that discriminates that the input image signal is a color image when the color difference signal is not less than the predetermined value.

In this construction, for example, a luminance signal (Y signal) component and color difference signal (U and V signal) components can be obtained by converting a RGB input image signal into a YUV signal, and it can be precisely discriminated whether the input image signal is a black-and-white image or a color image on the basis of the ratio of the color signal components.

Furthermore, in the image display device according to the first aspect of the invention, the black-and-white image processing unit may include a black and white discriminating unit that discriminates whether each sub-pixel in the predetermined unit of input image signal is a white pixel or a black pixel on the basis of the luminance values of the sub-pixels in the predetermined unit of the input image signals, and a line segment discriminating unit that compares the pattern of white pixels and black pixels contained in the predetermined unit of the input image signals to prestored segment patterns and discriminates that the predetermined unit of the input image signals is a line segment part when the pattern of white pixels and black pixels matches one of the prestored segment patterns. Therefore, it is possible to easily detect line segments by matching every predetermined unit of the input image with prestored segment patterns.

In addition, in the image display device according to the first aspect of the invention, the input image signal may contain pixel values as twice as the number of pixels of the display panel in the horizontal direction and the vertical direction, and the image processing units each may generate a color signal of one sub-pixel on the basis of the values of a plurality of sub-pixels that have the same color as the sub-pixel and are adjacent to the sub-pixel.

According to a second aspect of the invention, an electronic apparatus is provided which includes the image display device according to the first aspect of the invention and which can perform color display with a broad color reproduction range and black-and-white display with high resolution.

Embodiments of the present invention will now be described by way of further example only and with reference to the accompanying drawings, wherein like numbers reference like elements.

Fig. 1 is a chromaticity diagram showing the color reproduction range of a color filter according to the invention.

Fig. 2A is an example of the construction of the color filter.

Fig. 2B is another example of the construction of the color filter.

Fig. 2C is an example of black-and-white image display of the color filter.

Fig. 2D is another example of the black-and-white image display of the color filter.

Fig. 2E is another example of the construction of the color filter.

Fig. 2F is another example of the black-and-white image display of the color filter.

Fig. 3 is a view showing examples of a line segment pattern of a black-and-white image.

Fig. 4A is a view illustrating an example of a color sub-pixel rendering method.

Fig. 4B is a view illustrating another example of the color sub-pixel rendering method.

Fig. 4C is a view illustrating another example of the color sub-pixel rendering method.

Fig. 4D is a view illustrating another example of the color sub-pixel rendering method.

Fig. 4E is a view illustrating another example of the color sub-pixel rendering method.

Fig. 5 is a block diagram schematically showing the construction of a display device according to a first embodiment of the invention.

Fig. 6 is a block diagram schematically showing the construction of an image processing unit.

Fig. 7 is a block diagram illustrating the function of the image processing unit.

Fig. 8 is a flowchart illustrating a display process of the image processing unit.

Fig. 9A is a view showing an example of the arrangement of color filters according to a second embodiment of the invention.

Fig. 9B is a view showing an example of line segment pattern that can be represented by the color filters according to the second embodiment of the invention.

Fig. 9C is a view showing another example of the arrangement of the color filters according to the second embodiment of the invention.

Fig. 9D is a view showing another example of the line segment pattern that can be represented by the color filters according to the second embodiment of the invention.

Fig. 10 is a view showing the construction of a liquid crystal display panel according to the invention

Fig. 11A is a view showing an example of an electronic apparatus according to the invention.

Fig. 11B is a view showing another example of the electronic apparatus according to the invention.

Hereinafter, a preferred embodiment according to the invention will be described with reference to the accompanying drawings. In the following description, a liquid crystal panel will be described as an example of an electro-optical panel according to the invention.

### Color filter

First, a color filter according to the invention will be described. In this embodiment, a four-color filter which has RGB regions used as a general color filter and additional color region is used. As additional color, cyan, white (achromatic color), yellow, and so on are conceivable but cyan (C) will be used in the following description.

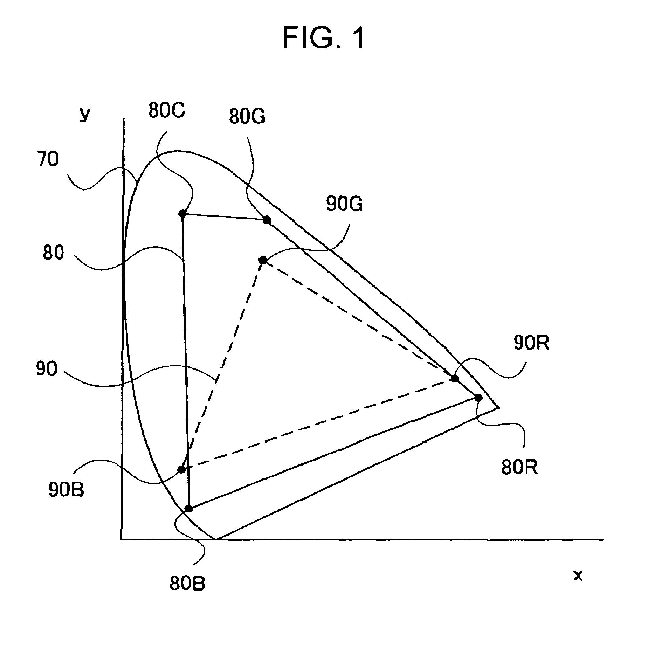

Fig. 1 shows a color reproduction area of a color filter on a CIE chromaticity diagram. A visible color region 70 for a human being is horseshoe-shaped as shown in Fig. 1. A triangular color reproduction area 90 shown by a dashed line is the color reproduction area of a RGB color filter and apexes 90R, 90G, and 90B correspond to red, green, and blue display colors, respectively. In other words, when using a RGB color filter, colors in the color reproduction area 90 are reproducible.

Meanwhile, the color reproduction area 80 of a four-color filter using cyan in addition to RGB has a rectangular shape shown by a solid line. Apexes 80R, 80G, 80B, and 80C correspond to red, green, blue, and cyan, respectively. As can be seen by comparing the color reproduction area 90 of a three-color filter shown in Fig. 1 with the color reproduction area 80 of a four-color filter, the color reproduction area that the display device can display is enlarged by using a four-color filter having a cyan color filter in addition to RGB color filters and thus the display device can display various colors.

### Black-and-white image display

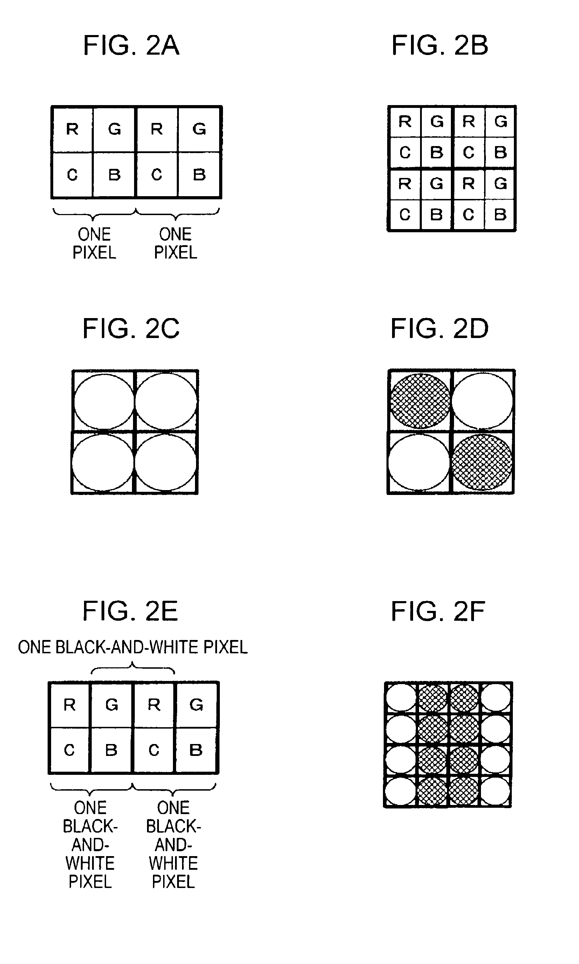

Next, the black-and-white image display of a display device using four-color filters will be described. Figs. 2A to 2F show examples of the construction of a four-color filter. As shown in Fig. 2A, one pixel is composed of R, G, B, and C sub-pixels. Now, in a case of considering that four pixels are arranged in a two-by-two matrix as shown in Fig. 2B and every pixel has R, G, B, and C color data, black-and-white images capable of being represented by the four pixels becomes pixel units as shown in Figs. 2C and 2D.

However, in a case of arrange two pixels in a horizontal direction, it is possible to form one pixel even by the combination of four sub-pixels in the middle, as shown in Fig. 2E. Therefore, when R, G, B, and C color data are prepared in each sub-pixel unit (that is, twice the number of the color pixels of the display panel in the horizontal direction and the vertical direction) and black or white is displayed by four sub-pixels in the middle of Fig. 2E, it is possible to improve the resolution into twice (twice in the horizontal direction in this example) as shown in Fig. 2F.

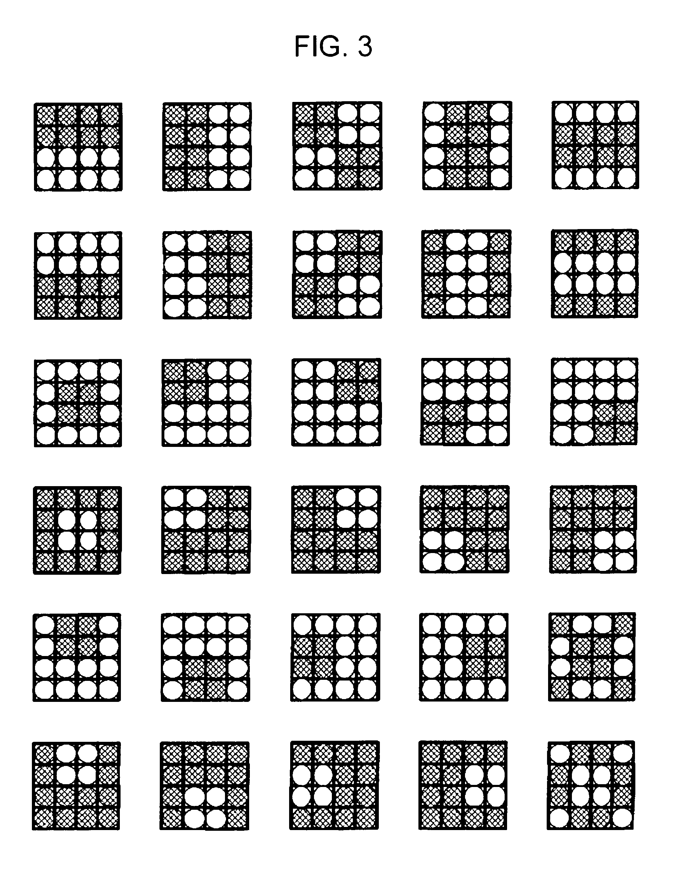

In this way, in black-and-white image display, it is possible to improve the resolution using the same four-color filters by performing display in units shifted by one sub-pixel (that is, half of a color pixel). Fig. 3 shows examples of a line segment pattern that can be displayed by two-by-two color pixels (that is, four by four sub-pixels) by the above-mentioned method.

When an input image is a black-and-white image, the image is generally configured by line segments such as characters or figures. Meanwhile, it is understood that the visual sensibility of a human being for a color image is high than that for a black-and-white image. Therefore, in the display device using a four-color filter according to the invention, when the input image is a black-and-white image, if line segments are detected from an input image signal and the line segments are displayed as black-and-white line segment patterns shown in Fig. 3, it is possible to improve the display resolution of a black-and-white image such as characters. Further, the detection of line segments is performed by matching the input image signals with the line segment patterns shown in Fig. 3, as will be described below.

### Color image display

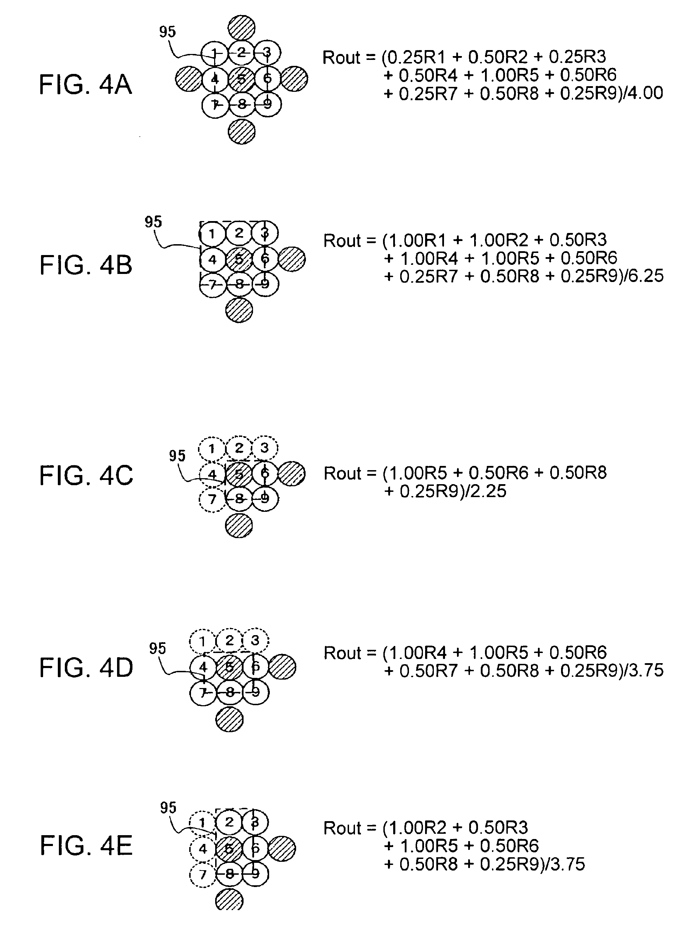

Next, color image display in the display device using a four-color filter will be described. When the input image is a color image and when the input image does not contain a line segment part even though the input image is a black-and-white image, the input image data is rendered to R, G, B, and C sub-pixels such that a color image is display (this is referred to as 'color-sub-pixel rendering'). Figs. 4A to 4E show methods of rendering color-sub-pixel.

In Figs. 4A to 4E, each circle corresponds to one sub-pixel. As the input image data, color data of twice as much as the number of pixels of the display panel in the horizontal direction and the vertical direction, respectively, as described above, that is, R, G, B, and C color data is input to every sub-pixel. Therefore, color data of one sub-pixel is calculated on the basis of color data of nine sub-pixels containing the one sub-pixel which have the same color.

In Figs. 4A to 4B, sub-pixels marked with diagonal lines are subjected to rendering and the value of sub-pixel denoted by reference numeral '5' is calculated here. In particular, the output value of one color pixel shown by a dashed line 95 is calculated on the basis of the values of the sub-pixels denoted by reference numerals '1' to '9' containing the color pixel. Further, in Figs. 4A to 4E, among R, G, B, and C color data, only R color data will be described but the same method can be applied to the other R, G, and B color data.

Fig. 4A shows the most typical example. Assuming that the pixel values of the sub-pixels denoted by reference numerals '1' to '9' are R1 to R9, respectively, the pixel value Rout of the color pixel shown by the dashed line 95 is calculated by Equation shown in Fig. 4A. In particular, the pixel values of each of the color pixels shown by the dashed line 95 is calculated by convoluting the pixel values of the sub-pixels in the region surrounded by the dashed line 95 using coefficients according to areas of the sub-pixels surrounded by the dashed line 95, respectively.

Fig. 4B shows a case in which a color pixel shown by a dashed line 95 locates at the upper left corner of one of image data, and Fig. 4C shows a case in which the central sub-pixel contained in a color pixel shown by a dashed line 95 locates at the upper left corner of one of image data. Further, Fig. 4D shows a case in which a color pixel shown by a dashed line 95 locates at the upper edge of one of image data, and Fig. 4E shows a case in which a color pixel shown by a dashed line 95 locates at the left edge of one of image data.

In this way, the rendering of each sub-pixel of the four-color filter is performed using an input image signal containing R, G, B, and C color data in every sub-pixel of the four-color filter (that is, an input image data having twice the resolution of color pixels composed of four-color filters in the horizontal direction and the vertical direction), thereby color image display can be performed in the broad color reproduction area as shown in Fig. 1. Further, in each of the examples of Fig. 4A to 4E, the pixel value of the color pixel shown by dashed line 95 is calculated by convoluting the pixel values of the sub-pixels in the color pixel surrounded by the dashed line 95 using coefficients according to areas of the sub-pixels contained in the color pixel, respectively. However, the pixel value of the color pixel may be convoluted using coefficients according to distances from the central sub-pixel.

### Image display device

### First embodiment

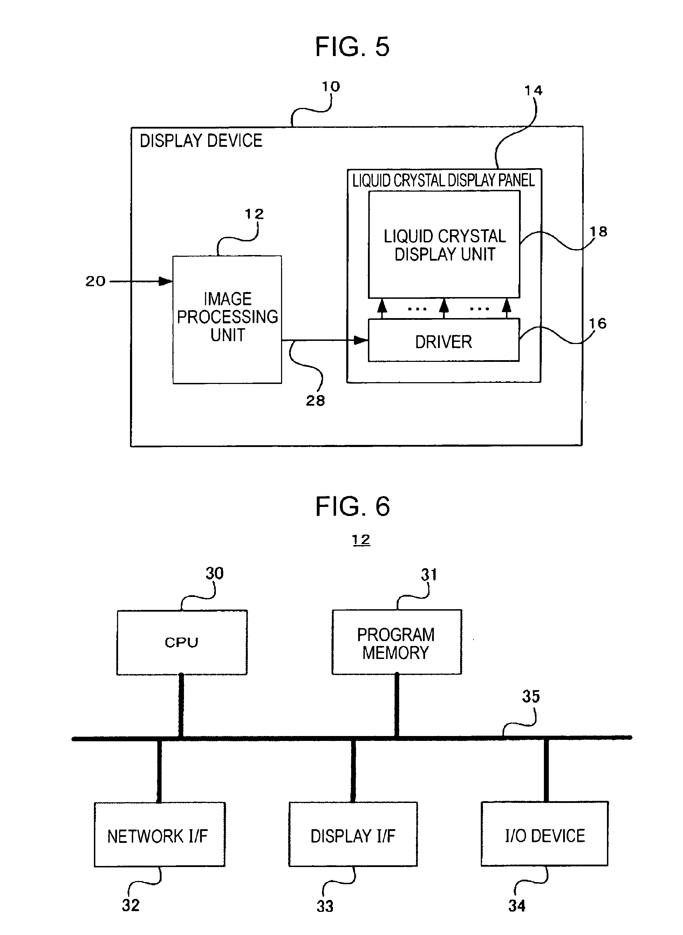

Next, a first embodiment of a display device to which the above-mentioned color filter is applied will be described. Fig. 5 shows an example of the construction of a display device 10 according to the first embodiment. The display device 10 can be applied to portable terminals such as a mobile phone, a PDA, etc. In Fig. 5, the display device 10 according to the first embodiment includes an image processing unit 12 and a liquid crystal display panel 14. The liquid crystal display panel 14 has a liquid crystal display unit 18 and a driver 16.

An RGB signal 20 is input from the outside to the display device 10. The RGB signal 20 includes an R signal Sr, a G signal Sg, and a B signal Sb. The image processing unit 12 generates four-color signals from the input RGB signal 20. The four-color signals correspond to R, G, B, and C, respectively, and are supplied to the driver 16 in the liquid crystal panel 14.

The liquid crystal display unit 18 is a liquid crystal display unit to which the above-mentioned four-color filter is applied. The driver 16 drives individual pixels of the liquid crystal display unit 18 on the basis of the input four-color signal. In this way, each pixel composed of a four-color filter is driven as shown in Fig. 2 and so on, and thus the image input as the RGB signal 20 is displayed in the liquid crystal display unit 18.

Next, the image processing unit 12 will be described in detail. The image processing unit 12 generates a four-color signal, which corresponds to each sub-pixel region of the four-color filter provided in the liquid crystal display unit 18, from the input RGB signal.

As has already been described, the four-color filter according to the embodiment of the invention has an advantage that the four-color filter has a broader color reproduction area for a color image signal than a general RGB color filter. Meanwhile, as has been described with reference to Figs. 2 and 3, it is possible to display line segments contained in a black-and-white image with improved resolution than that of a color pixel.

Further, in this embodiment, it is discriminated whether the input image is a black-and-white (achromatic) image or a color (chromatic) image and then different processes are performed on the individual images. In particular, when the input image is a black-and-white image, line segments (vertical lines and horizontal lines) are detected from the input image and white or black is assigned to every pixel, thereby performing display that accentuates line segments (hereinafter, referred to as a 'lining process'). In this way, when the input image is a text or the like, it is possible to clearly display characters, figures, and so on.

Meanwhile, when the input image is a color image, the color image is displayed by the above-mentioned color sub-pixel rendering.

Fig. 6 is a block diagram schematically showing the construction of the image processing unit 12 in a case of performing color conversion into the four-color filter by using software. The image processing unit 12 is configured such that a CPU 30, a program memory 31, a network I/F 32, a display I/F 33, an I/O device 34 are connected to a bus 35. The program memory 31 stores a display process program to be described later. The network I/F 32 is used in a case of obtaining a source image such as an RGB signal or the like from a network, etc. The display I/F 33 is an interface for supplying four color signals 28 obtained by image processing to the liquid crystal display panel 14. The I/O device 34 is a device used for a user to do selection/instruction containing source image selection and so on. The CPU 30 not only controls the components of the image processing unit 12 but also performs display processing to be described later by executing a display process program stored in the program memory 31.

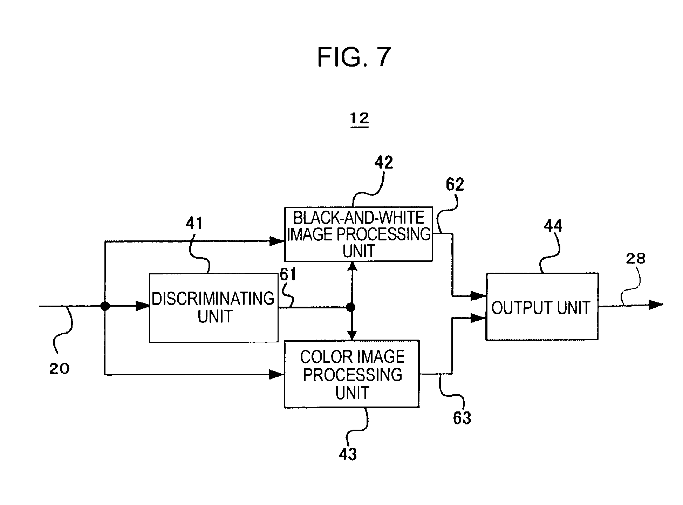

Fig. 7 is a block diagram illustrating the function of the image processing unit 12. The image processing unit 12 functionally has a discriminating unit 41, a black-and-white image processing unit 42, a color image processing unit 43, and an output unit 44. These components are implemented such that the CPU 30 executes a predetermined program, which is stored in the program memory 31.

The RGB signal 20 input to the image processing unit 12 is input to the discriminating unit 41, the black-and-white image processing unit 42, and the color image processing unit 43. The discriminating unit 41 converts the RGB signal 20 into a YUV signal and then generates a luminance signal Y and color difference signals U and V. Subsequently, the discriminating unit 41 discriminates whether the input RGB signal is a black-and-white image or a color image on the basis of the obtained color difference signals. In particular, the discriminating unit 41 discriminates whether the color difference signals U and V are less than a predetermined value X. When both signals are less than the predetermined value X, the discriminating unit 41 discriminates that the input image is a black-and-white image, and when at least one of both signals is not less than the predetermined value X, the discriminating unit 41 discriminates that the input image is a color image. As the predetermined value X, for example, a value around '0.1' (that is, 10%) can be used. In this case, the discriminating unit 41 discriminates that an image of which color component is less than 10% is a black-and-white image and an image of which color component is not less than 10% is a color image. A discrimination result signal 61 obtained in this way is sent to the black-and-white image processing unit 42 and the color image processing unit 43.

When the discrimination result signal 61 represents that the input image is a black-and-white image, the black-and-white image processing unit 42 operates to generate an image signal 62 by performing a lining process to be described on the input image so as to accentuate lines of the input image, and sends the generated image signal to the output unit (γ converter) 44 when the discrimination result signal 61 represents that the input image is a black-and-white image. Meanwhile, when the discrimination result signal 61 represents that the input image is a color image, the color image processing unit 43 operates to generate an image signal 63 by performing the color sub-pixel rendering process on the input image so as to improve the resolution, and sends the image signal 63 to the output unit 44. The output unit 44 performs γ conversion on the supplied image signal 62 or 63 on the basis of a predetermined γ characteristic to output the converted result as four color signals 28.

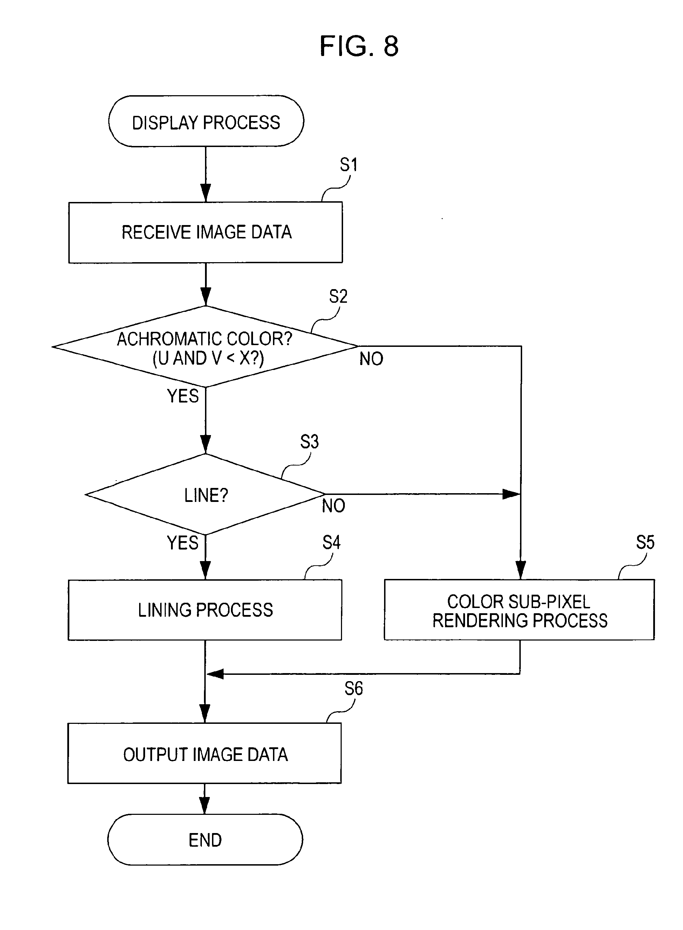

Fig. 8 is a flowchart of display process performed by the image processing unit 12. When the CPU 30 executes the image display program so as to implement the function of the components shown in Fig. 7 as described above, the display process is performed. First, the discriminating unit 41 receives image data (that is, a RGB signal 20) from the outside (step S1). Next, the discriminating unit 41 generates color difference signals U and V by converting the image data into a YUV signal and discriminates whether the input image is a black-and-white (achromatic) image or a color (chromatic) image by comparing the color difference signals U and V to a predetermined value X (step S2).

Further, the discriminating unit 41 discriminates whether image data is a black-and-white image or a color image for every predetermined unit of image data. The predetermined unit of image data can be, for example, a two-by-two color pixels (that is, a four-by-four sub-pixels) shown in Fig. 2 or 3. The input image data has generally a resolution that is twice the resolution of color pixels in the horizontal direction and the vertical direction, respectively. In other words, the input image data has RGB color data for every sub-pixel. Therefore, the discriminating unit 41 performs YUV conversion on every sub-pixel of the predetermined unit of image data composed of the four-by-four (total sixteen) sub-pixels and discriminates whether each sub-pixel is a black-and-white sub-pixel or a color sub-pixel on the basis of the color difference signals U and V. When at least one of the sixteen sub-pixels is a color sub-pixel, the discriminating unit 41 discriminates that the predetermined unit of image data is a color image. When all the sixteen sub-pixels are black-and-white sub-pixels, the discriminating unit 41 discriminates that the predetermined unit of image data is a black-and-white image.

When it is discriminated that the input image is a black-and-white (Yes in step S2), the black-and-white image processing unit 42 detects lines. In order to detect lines, for the predetermined unit of image data (four-by-four sub-pixels in this embodiment), it is discriminated whether each sub-pixel is a black-and-white sub-pixel or a color sub-pixel by using a predetermined threshold. When it is discriminated that all the sixteen sub-pixels are white or black sub-pixels and the pattern matches any one of the line segment patterns shown in Fig. 3, it is discriminated that the predetermined unit of image data is a line segment part (Yes in step S3). Meanwhile, when the four-by-four sub-pixels includes a sub-pixel (that is, a gray pixel) other than white sub-pixels and black sub-pixels or when the pattern of white sub-pixels and black sub-pixels does not match any one of the line segment patterns shown in Fig. 3, it is discriminated that the predetermined unit of image data is not a line segment part (No in step S3).

When it is discriminated that the predetermined unit of image data is a line segment part (Yes in step S3), the black-and-white image processing unit 42 substitutes the predetermined unit of image data with the line segment pattern which matches the predetermined unit of image data. In other words, the black-and-white image processing unit 42 substitutes the color data of each sub-pixel of the predetermined unit of image data with the white sub-pixel value or the black sub-pixel value of the line segment pattern of Fig. 3 corresponding to the predetermined unit of image data. In this way, it is possible to improve the resolution of the line segment part. Subsequently, the black-and-white image processing unit 42 outputs the color data obtained by the substitution to the liquid crystal display panel 14 through the output unit 44 such that display is performed on the liquid crystal display panel 14 (step S6).

Meanwhile, when it is discriminated that the input image is a color image (No in step S2) or when it is discriminated that the predetermined unit of image data is not a line segment part even though it is discriminated that the input image is a black-and-white image (No in step S3), the color image processing unit 43 generates color image data by performing the color sub-pixel rendering process described above with reference to Fig. 4 and supplies the generated color image data to the liquid crystal display panel 14 (step S6). At the time of performing the color sub-pixel rendering process, the color image processing unit 43 coverts the YUV image data obtained in step S2 into RGBC image data. This converting process can be performed, for example, by using a three-dimensional look-up table (LUT) defining the correspondence relation between YUV values and RGBC values, etc.

As described above, in the display device having four-color filters according to the first embodiment of the invention, when the input image is a black-and-white image and has line segments, four-color data is defined by a lining process so as to accentuate the line segments. Therefore, it is possible to improve the resolution of the black-and-white image. Meanwhile, when the input image is a color image and when the input image is a black-and-white image but the input image doesn't have any line segment, four-color data is generated by the color sub-pixel rendering process. Therefore, it is possible to perform color image display with excellent color reproductivity.

### Second embodiment

Even though a second embodiment is basically same as the first embodiment, the resolution is improved in the horizontal or vertical direction by devising arrangement of sub-pixels in four-color filters.

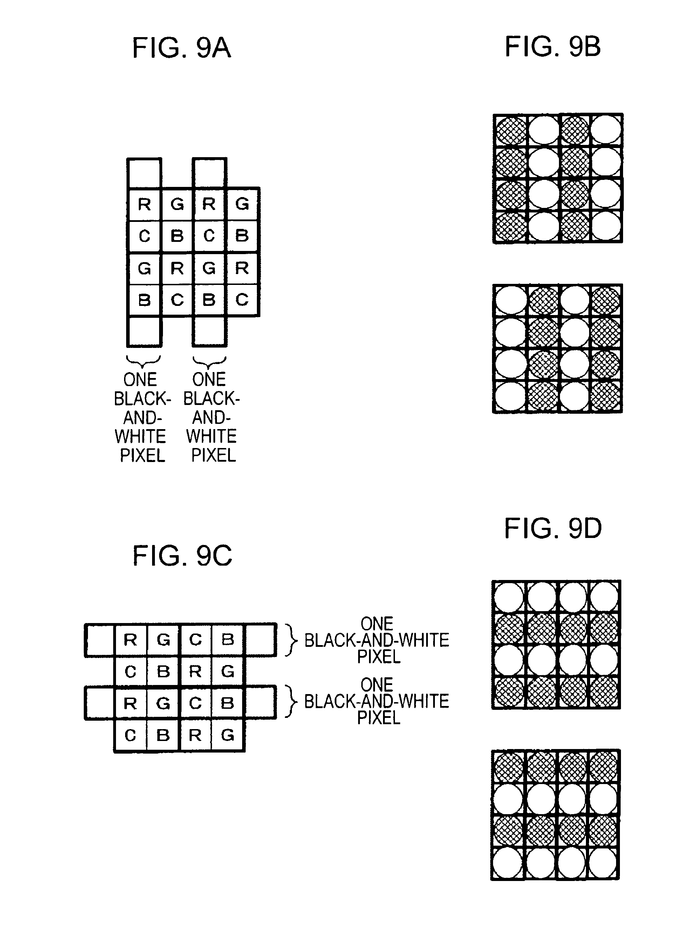

Fig. 9A shows an example of the arrangement of sub-pixels of four-color filter with improved resolution in the horizontal direction. In the example of Fig. 9A, four sub-pixels arranged in the vertical direction can constitute one black-and-white pixel. Therefore, line segment patterns shown in Fig. 9B also can be used, thereby it is possible to further improve the resolution of a black-and-white image in the horizontal direction.

Fig. 9C shows another example of the arrangement of sub-pixels of four-color filter with improved resolution in the vertical direction. In the example of Fig. 9C, four sub-pixels arranged in the horizontal direction can constitute one black-and-white pixel. Therefore, line segment patterns shown in Fig. 9D also are used, thereby it is possible to further improve the resolution of a black-and-white image in the vertical direction.

### Liquid crystal display panel

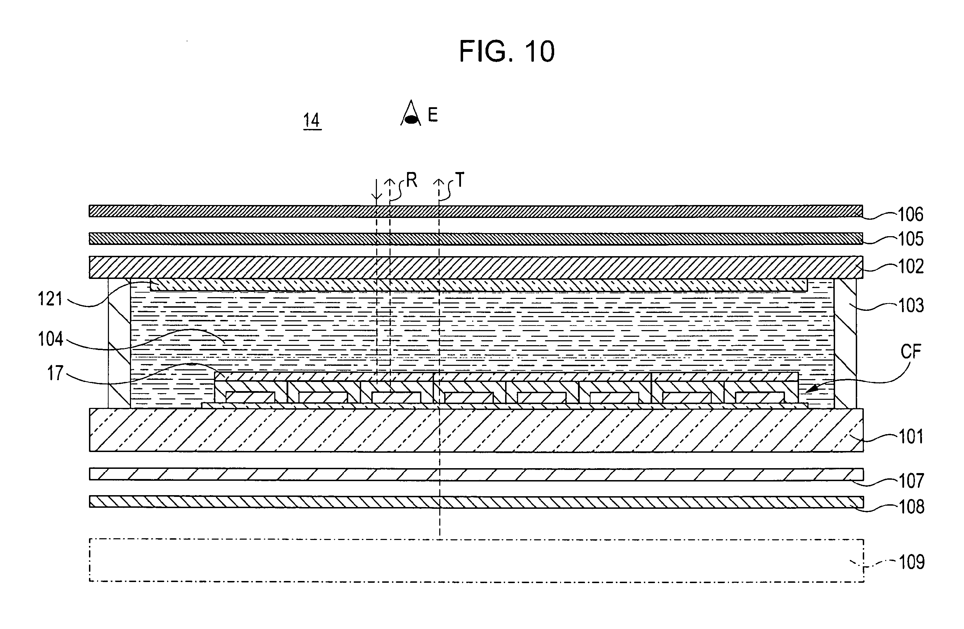

Next, an example of a liquid crystal display panel to which a color filter substrate according to the invention is applied will be described. According to this example, a color filter substrate having the above-mentioned four-color filters is applied to a transflective liquid crystal display panel 14. Fig. 10 is a cross-sectional view of the liquid crystal display unit 18.

In Fig. 10, the liquid crystal display panel 14 includes two substrates 101 and 102 that are made of glass, plastic, or the like and are bonded to each other by means of a sealant 103, and liquid crystal 104 interposed between the substrates 101 and 102. Further, a retardation film 105 and a polarizing plate 106 are subsequently disposed on the external surface of the substrate 102, and a retardation film 107 and a polarizing plate 108 are subsequently disposed on the external surface of the substrate 101. Furthermore, a backlight 109 which emits illuminating light at the time of performing transmissive display is disposed below the polarizing plate 108.

The substrate 101 is a transparent substrate made of glass or the like and the above-mentioned four-color filters CF are formed on the substrate 101. In particular, R, G, B, and C filter regions are arranged as described above. Further, if necessary, a transparent resin scattering layer may be formed of an acryl resin or the like on the substrate 101. Furthermore, metal films may be formed in reflective regions on the resin scattering layer. In addition, in the reflective regions, color filters may be formed on the metal films, respectively.

Furthermore, if necessary, a black matrix may be formed at borders among the individual color filters. On the color filters CF, transparent electrodes 17 are formed of a transparent conductive material such as ITO (indium tin oxide). According to the present embodiment, the transparent electrodes 17 are formed in stripes to be parallel to each other. Also, the transparent electrodes 17 extend in the direction orthogonal to transparent electrodes 121 which are formed on the substrate 102 in stripes. The members that constitute the liquid crystal display panel 100 and are included at intersections between the transparent electrodes 17 and the transparent electrodes 121 constitute pixel regions 20.

On the other hand, transparent electrodes 121 are formed on the internal surface of the substrate 102 so as to intersect the transparent electrodes 17 on the substrate 101 opposite to the substrate 102. Further, if necessary, alignment films may be formed on the transparent electrodes 17 on the substrate 101 and on the transparent electrodes 121 on the substrate 102.

In the liquid crystal display panel 14, when the reflective display is performed, external light incident onto the region where the metal reflecting films are formed is directed along the path R illustrated in Fig. 10 and is reflected by the metal reflecting films so that an observer can view the external light. On the other hand, when the transmissive display is performed, the illuminating light emitted from the backlight 109 is incident onto the transmissive region and travels along the path T so that an observer can view the illuminating light.

The above-mentioned liquid crystal display panel is just an example in which the four-color filter according to the invention is applied, and the four-color filter according to the invention can be applied to various liquid crystal display panels having other constructions.

### Electronic apparatus

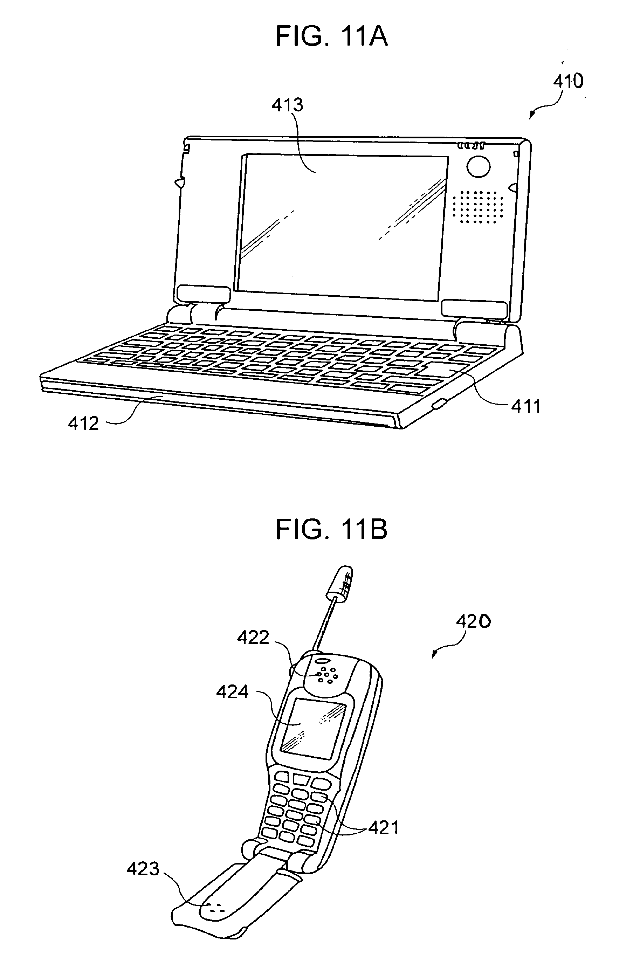

Next, an example of an electronic apparatus to which the liquid crystal display panel according to the invention can be applied will be described with reference to Figs. 11A and 11B.

First, an example in which the liquid crystal display panel according to the invention is applied to a display unit of a portable personal computer (a so-called notebook personal computer) will be described. Fig. 11A is a perspective view showing the construction of the personal computer. As shown in Fig. 11B, a personal computer 410 includes a main body 412 having a keyboard 411 and a display unit 413 to which the liquid crystal display panel according to the invention is applied.

Subsequently, an example in which the liquid crystal display panel according to the invention is applied to a display unit of a mobile phone will be described. Fig. 11B is a perspective view showing the construction of the mobile phone. As illustrated in Fig. 11B, a mobile phone 420 includes a plurality of operating buttons 421, an earpiece 422, a mouthpiece 423, and a display unit 424 to which the liquid crystal display panel according to the invention is applied.

In addition, the electronic apparatuses to which the liquid crystal display panels according to the invention can be applied include a liquid crystal TV, a view finder type and monitor direct-view-type videotape recorder, a car navigator, a pager, an electronic organizer, a calculator, a word processor, a work station, a video phone, a POS terminal, and a digital still camera, as well as the personal computer shown in Fig. 11A and the mobile telephone shown in Fig. 11B.

The substrate and the liquid crystal device having the above-mentioned reflective layer and color filters are not limited to the above-mentioned embodiments but various changes may be made without departing from the scope of the invention.

According to the above-mentioned embodiments, the liquid crystal display panel is described as an example. However, the electro-optical device according to the invention can also be applied to an electrophoresis device such as an electronic paper and an electroluminescent (EL) device.

## Claims

1. An image display device comprising:
a display panel in which each pixel region has four color sub-pixel regions,
an image processing unit that generates color signals for the four color sub-pixels on the basis of an input image signal from the outside, and
a control unit that display an image in the pixels of the display panel on the basis of the color signals,
wherein the image processing unit includes:
a discriminating unit that discriminates whether the input image signal is a black-and-white image or a color image for every predetermined unit of the input image signals;
a black-and-white image processing unit that detects line segment parts forming line segments from the input image signal and generates color signals for a black-and-white image corresponding to the line segment parts when it is discriminated that the input image signal is a black-and-white image; and
a color image processing unit that generates color signals for a color image when it is discriminated that the input image signal is a color image and when it is discriminated that the predetermined unit of the image data is not the line segment parts.

2. The image display device according to Claim 1,
wherein the regions of the sub-pixels of four colors are arranged in a vertical direction so as to form one vertical pixel region, and
the black-and-white image processing unit generates the color signals for a black-and-white image representing white or black in one pixel unit in the vertical direction.

3. The image display device according to Claim 1,
wherein the regions of the sub-pixels of four colors are arranged in a horizontal direction so as to form one horizontal pixel region, and
the black-and-white image processing unit generates the color signals for a black-and-white image representing white or black in one pixel unit in the horizontal direction.

4. The image display device according to Claim 1,
wherein the discriminating unit includes:
a converting part that converts the input image signal into a luminance signal and a color difference signal for every predetermined unit; and
a discriminating part that discriminates that the input image signal is a black-and-white image when the color difference signal is less than a predetermined value and that discriminates that the input image signal is a color image when the color difference signal is not less than the predetermined value.

5. The image display device according to Claim 1,
wherein the black-and-white image processing unit includes:
a black and white discriminating unit that discriminates whether each sub-pixel in the predetermined unit of input image signal is a white pixel or a black pixel on the basis of the luminance values of the sub-pixels in the predetermined unit of the input image signals; and
a line segment discriminating unit that compares the pattern of white pixels and black pixels contained in the predetermined unit of the input image signals with prestored segment patterns to discriminate that the predetermined unit of the input image signals is a line segment part when the pattern of white pixels and black pixels matches one of the prestored segment patterns.

6. The image display device according to Claim 1,
wherein the input image signal has a pixel value as twice as the number of pixels of the display panel in the horizontal direction and the vertical direction, and
the image processing units each generate a color signal of one sub-pixel on the basis of the values of a plurality of sub-pixels that have the same color as the sub-pixel and are adjacent to the sub-pixel.

7. An electronic apparatus comprising:
the image display device according to Claim 1 as an image display unit.
